# EUROPEAN PATENT SPECIFICATION

(11) **EP 1 493 450 B1**
(45) Date of publication and mention of the grant of the patent: **15.04.2009**
(21) Application number: 04253890.0
(22) Date of filing: 29.06.2004
(51) Int. Cl.: A61L 2/26, B01J 3/03, F16J 13/18

(54) **Sterilization vacuum chamber door closure**
Türschliessvorrictung für einen Vakuumsterilisator
Dispositif de fermeture de porte d'un stérilisateur sous vide

(30) Priority: 30.06.2003 US 609639
(43) Date of publication of application: 05.01.2005
(73) Proprietor: ETHICON, INC., Somerville, NJ 08876 (US)
(72) Inventor: Morrison, Todd, Dana Point, CA 92629 (US)
(74) Representative: Fisher, Adrian John

(56) References cited:
- US-A- 2 352 192
- US-A- 3 144 956
- US-A- 5 313 738
- US-B1- 6 391 258

## Description

### BACKGROUND OF THE INVENTION

The present invention relates to a sterilization vacuum chamber and a door closure therefor.

When closing a door to a sterilization chamber which will be put under vacuum, it is desirable to distribute forces evenly to avoid leaks or damaging a seal between the door and chamber. A typical closure comprises hinges at one side of the door and a latch at the other. When the latch pulls the door closed, unless the hinges and latch are precisely placed forces between the door and chamber will be higher on one side of the door than the other. This can lead to leaks and damage seals between the door and chamber and also damage the hinges.

US 5,313,738 discloses a vacuum sterilization chamber of the type defined in the precharacterizing portion of the accompanying claim 1.

US 3,144,956 discloses a vacuum sterilization chamber having a biasing spring in cooperation with a sliding hinge to urge a door to a closing position.

### SUMMARY OF THE INVENTION

A vacuum sterilization chamber has a sealable doorway. The sealable doorway according to the present invention comprises an opening into the chamber and a door for covering the opening. A hinge connects the door to the chamber and a latch releasably connects the door to the chamber. A force equalization system at the hinge or at the latch comprises the door having a degree of movement toward and away from the chamber and a biasing member urging the door toward the chamber.

Preferably, the force equalization system is at the hinge. The hinge can slidably connect to the chamber thereby to allow the degree of movement toward and away from the chamber. A low friction sliding surface is preferably provided to assist this movement, such as placing the hinge between two low friction plates formed of or coated with PTFE. Preferably, the hinge attaches via a fastener through an elongated slot on the hinge.

Alternatively, the hinge can slidably connect to the door so as to allow the degree of movement toward and away from the chamber.

Preferably, the biasing member is a spring. The biasing member could be anything which applies a force such as a counterweight, electromagnet, etc.

Preferably, there is a seal about the opening between the door and the chamber.

Preferably, the latch and hinge are on or near opposite sides of the opening, or at least spaced apart sufficiently to allow the forces to normalize.

A method of sealing a door to a vacuum sterilization chamber at an opening thereinto comprises the steps of:
closing the door about a hinge between the door and the chamber;
latching the door to the chamber with a latch; and
at either the latch or the hinge, allowing a degree of freedom of the door toward and away from the chamber and biasing the door toward the chamber.

Preferably, the door is allowed a degree of freedom toward and away from the chamber at the hinge, as by allowing the hinge to slide relative to the chamber. Such movement can be provided by attaching the hinge to the chamber via screws through elongated slots through the hinge. The hinge can attach to the chamber via a mounting bracket attached to the chamber.

A biological indicator evaluator resistomer vessel is known in the art as a 'BIER' vessel.

### BRIEF DESCRIPTION OF THE DRAWINGS

FIG. 1 is a block diagram of a BIER vessel according to the present invention;
FIG. 2 is a perspective view of the BIER vessel of FIG. 1;
FIG. 3 is a detailed perspective view of a latch mechanism on the BIER vessel of FIG. 1;
FIG. 4 is cross-sectional view taken along lines 4--4 of FIG. 2 showing a spring-loaded floating hinge;
FIG. 5 is an exploded perspective view of an alternative spring-loaded floating hinge mechanism;
FIG. 6 is an exploded perspective view of a mounting bracket of the hinge mechanism of FIG. 5;
FIG. 7 is a side elevation view of a hinge attachment mechanism of the hinge mechanism of FIG. 5;
FIG. 8 is a perspective view of a sample rack for use within the BIER vessel of FIG. 1; and
FIG. 9 is a perspective view of an alternative sample rack for use within the BIER vessel of FIG. 1

### DETAILED DESCRIPTION

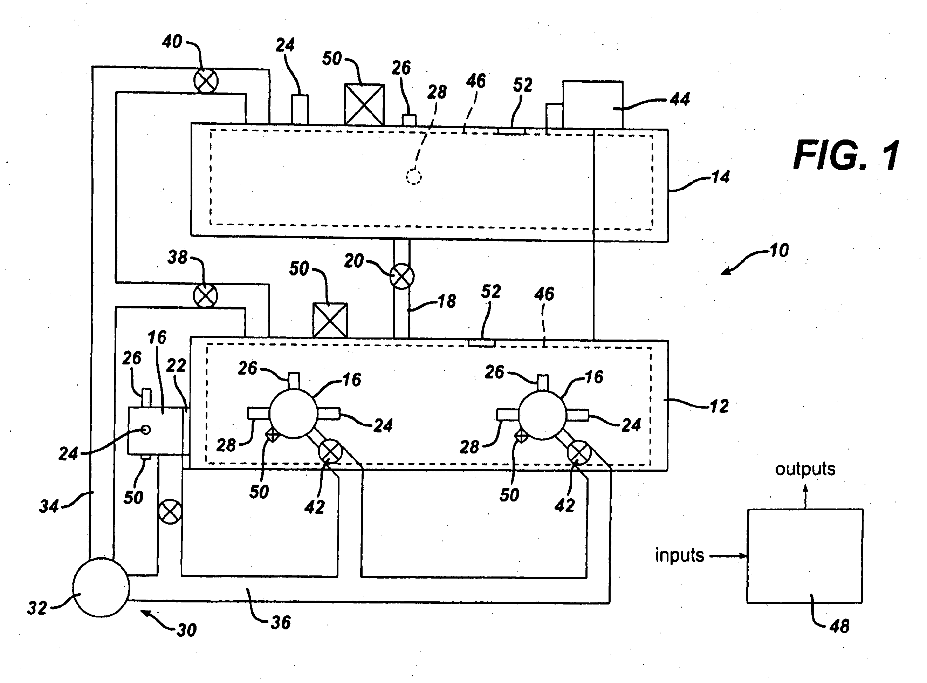

Fig 1. discloses in block diagram format an improved BIER vessel 10 according to the present invention. The BIER vessel 10 comprises a first chamber 12 typically employed as a vaporization chamber and a second chamber 14 typically employed as a test chamber. In this example the chambers 12 and 14 are of similar size, however their sizes can be varied to accommodate individual needs. A plurality of test chambers 16 attach to the vaporization chamber 12. These test chambers 16 are much smaller in size then the vaporization chamber 12 whereby upon placing the test chamber 16 into communication with the vaporization chamber 12 the conditions of the vaporization chamber 12 are quickly established within the test chamber 16 to provide an accurate starting point for a test. The most desirable starting point in a test would have the concentration of vaporized sterilant in the test chamber 16 change instantaneously from zero to the desired test concentration. A conduit 18 connects the first chamber 12 and second chamber 14 and incorporates an isolation valve 20 to separate the first chamber 12 from the second chamber 14. Similarly, each of the test chambers 16 are isolated from the first chamber 12 by an isolation valve 22.

Monitoring of conditions within the BIER vessel system 10 helps assure that the process is proceeding as desired. A separate pressure monitor 24, temperature sensor 26 and sterilant concentration monitor 28 is provided for each of the first chamber 12, second chamber 14 and test chambers 16. Sterilant monitors for hydrogen peroxide preferably employ light absorption techniques, such as described in the Prieve et al. U.S. Patent No. 6,269,680.

A vacuum system 30 comprises vacuum pump 32 and vacuum lines 34 from the vacuum pump 32 to the first and second chambers 12 and 14 and vacuum lines 36 serving the smaller test chambers 16. Vacuum vent valves 38 and 40 on the first chamber and second chamber 12 and 14, respectively isolate these chambers from the vacuum line 34 and vacuum vent valves 42 isolate the smaller test chambers 16 from the vacuum lines 36. The volume of the vacuum lines 36 exceeds the volume of their associated test chamber 16 such that upon opening the vent valves 42 contents of the test chamber 16 are quickly evacuated. When this occurs at the end of an exposure period to a sterilant, the concentration of sterilant in the test chamber 16 is quickly diminished so as to provide a controllable end point the exposure period. Similar to the starting point, the most desirable end point would have the sterilant concentration drop from the desired testing concentration to zero instantaneously.

A plasma generator 44 connects to electrodes 46 in the first and second chambers 12 and 14 provide the capability of driving the gases therein into the plasma state. The electrodes 46 are isolated from their respective chambers 12 and 14 and the plasma generator 44 applies an electrical potential between the electrode 46 and the respective test chamber 12 or 14. Examples of appropriate plasma generation systems are described in US Patents Nos. 4,801,421, 5,656,238 and 6,447,719.

A control system 48 interconnects to the various sensors 24, 26, 28, valves 20, 22, 38, 40, 42, the plasma generator 44 and the vacuum system 30 and other equipment as may be needed or desired to affect control over the process of the BIER vessel 10. Preferably, the control system includes data storage and networking capabilities for easy handling of the test data.

Vent valves 50 are provided on each of the chambers, 12, 14 and 16 to allow venting of the chamber to atmospheric pressure or a target pressure below atmospheric. These vent valves 50 are also connected to, and are under the control of the control system 48. Preferably they comprise a dual valve couple, one being larger than the other, to provide quick venting of large volumes and fine tuning of desired pressure. They are cycled open and closed until the target pressure is reached. A separate injector 52 is provided for first chamber 12 and second chamber 14, through which a premeasured quantity of liquid sterilant solution can be injected via a syringe through a septum and then vaporized into the chamber 12 or 14.

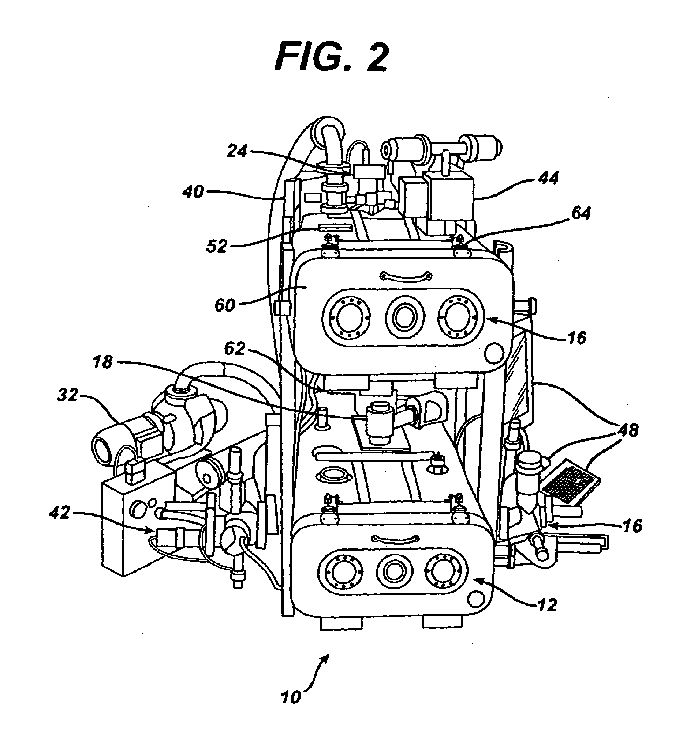

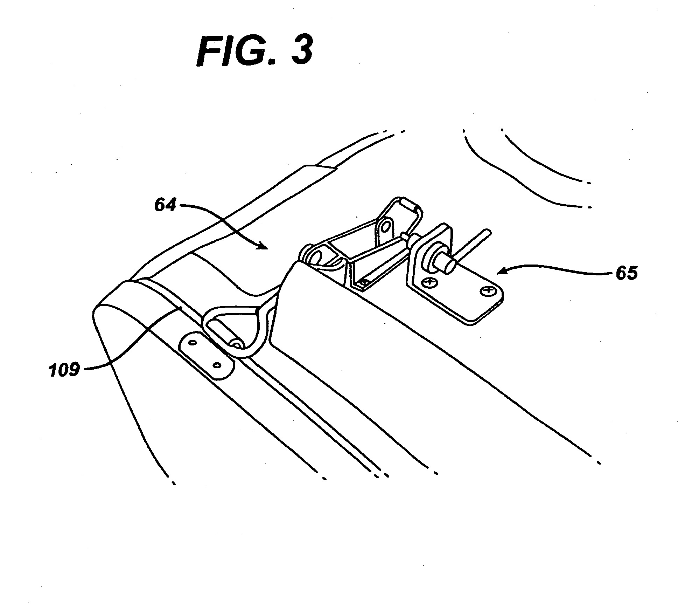

FIG. 2 shows in perspective view the BIER vessel 10 depicted in block diagram form in FIG. 1. Each of chambers 12 and 14 has a large door 60 having a floating hinge mechanism 62 and interlocking latch 64. The latch 64 is connected to the control system 48 and plasma generator 44 to extinguish the plasma if the door 60 is opened during the cycle when there is plasma present or when the concentration of sterilant is too high. As also seen in FIG. 3, a pneumatic piston 65, under control of the control system 48, extends over the latch 64 to prevent opening of the latch during unsafe conditions.

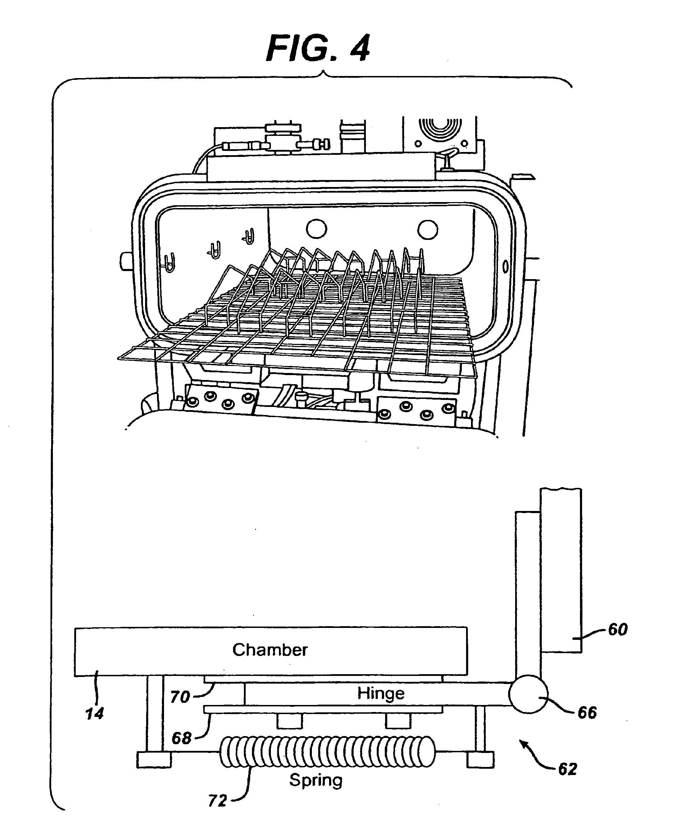

FIG. 4 shows the hinge mechanism 62 which comprises a hinge 66 attached to the door 60 and slideably attached to the outer wall of the chamber 12 or 14. The hinge 66 is trapped between an outer plate 68 and an inner plate 70 while retaining freedom to slide therebetween. Preferably, the plates 68 and 70 are formed out of or coated with a low friction substance such as polytetrafluoroethylene (PTFE). A spring 72 biases the hinge toward the chamber 12 or 14. When the door 60 is closed, the spring normalizes the forces applied at the top and bottom of the chamber 12 or 14.

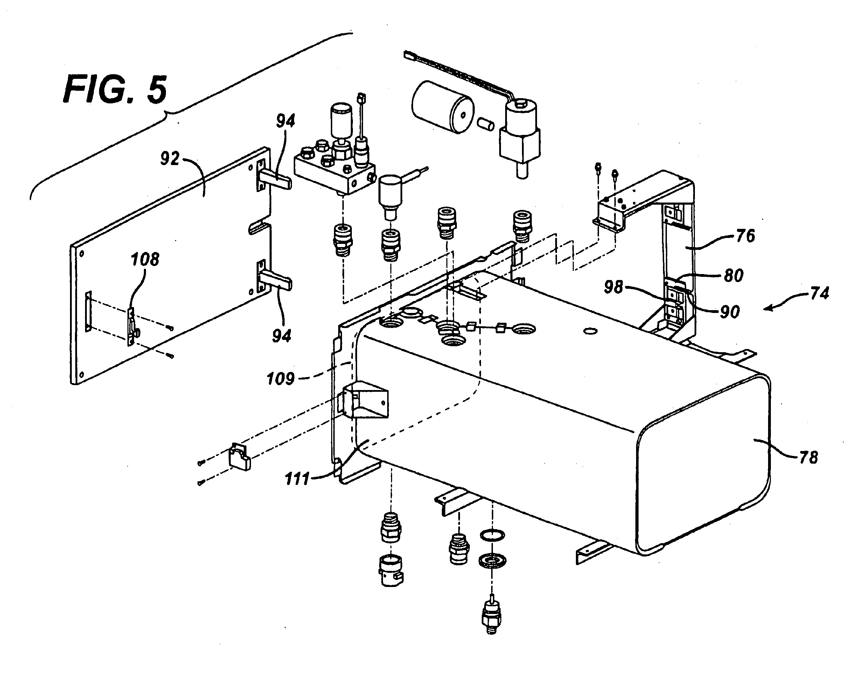

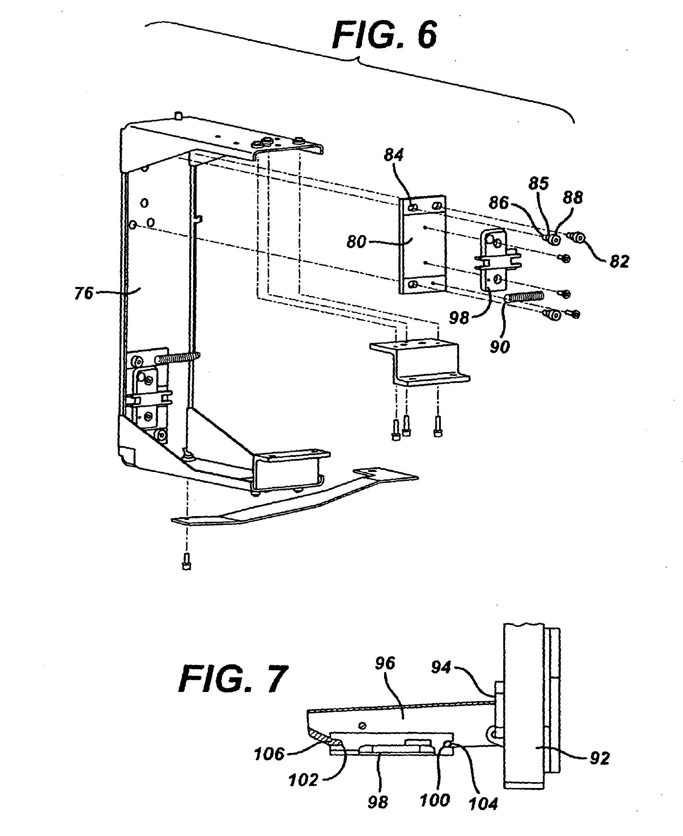

FIGS. 5 to 7 show an alternative version hinge mechanism 74. A hinge mounting bracket 76 affixes to a chamber 78. Two mounting plates 80 connect to the bracket 76 via screws 82 passing through elongated slots 84 in the mounting plate 80 so as to provide a limited degree of lateral movement of the plate 80 relative to the bracket 76. The screws 82 comprise an unthreaded shoulder 85 between machine threads 86 and a head 88 to allow easy movement of the shoulder 85 within the slots 84. A spring 90 biases the mounting plate 80 away from a door 92.

Two hinges 94 attach to the door 92 and have connectors 96 extending therefrom toward the mounting plates 80. A clip plate 98 attaches to each mounting plate 80 and has rear notches 100 and front notches 102 into which snap respectively a proximal pin 104 and distal catch 106 on the connectors 96 thus allowing easy attachment and detachment of the door 92. A latch 108 is provided on a side of the door 92 opposite the hinges 94. A seal 109 about an opening 111 into the chamber 78 and between the door 92 and the chamber 78 helps preserve a vacuum in the chamber 78.

Although the hinges 94 are shown slidably attached to the chamber 78, one of skill in the art would see that their design could be modified to slidably attach the hinges 94 to the door 92. Further, rather than allow movement at the hinges to normalize forces on the door seal 109, movement could instead be provided at the latch 108 or at both the latch 108 and the hinges 94.

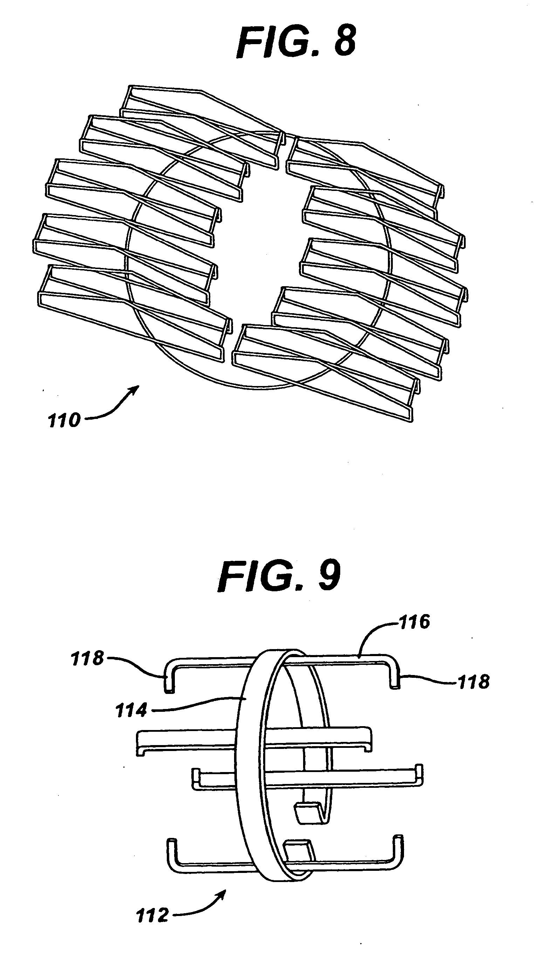

Test racks for holding biological indicators are helpful in getting even distribution of the indicators within the test chambers 14 and 16. FIG. 8 shows a rack 110 suitable for holding twenty flat test packs (not shown), each containing a biological indicator, for use within the large test chamber 14. FIG. 9 shows a rack 112 suitable for holding four biological indicators within the cylindrical test chamber 16. An open circular ring 114 having evenly spaced holder 116 about its circumference fits snugly within the test chamber 16. Each holder 116 has a pair of end flanges 118 between which can be placed a biological indicator.

A typical cycle in the BIER vessel 10 comprises the following.

Heat all portions of the BIER Vessel 10 to 50° C. OPTIONALLY: Heat the vaporizer chamber 12 to 65° C, the large test chamber 14 to 50° C, and the small test chambers or ports 16 to 45° C. The temperature differentials cause small pressure gradients in the gas which can be manipulated to help control gas flow.

Evacuate all portions of the BIER Vessel to 0.2 Torr and light a plasma. The vacuum and plasma both aid in eliminating any possible residuals, such as water or sterilant from a prior cycle, in the vessel. As plasma energy creates heat, the pressure may rise. The 0.2 Torr pressure can be maintained with the throttling valves 50 that opens and closes to raise or lower pressure.

Vent all chambers 12, 14 and 16 to atmospheric pressure and load samples. Samples are preferably positioned within all portions of the chamber 14 so that when sterilant is introduced into the portions, it exposes all samples equally.

Evacuate all portions of the BIER Vessel 10 to 0.2 Torr. The vacuum enhances sterilant vaporization and diffusion. OPTIONALLY: Plasma may be introduced to condition/heat the samples/load. Stop the plasma at the end of the conditioning time.

Close respective valves to isolate all portions of the BIER vessel 10 at the 0.2 Torr pressure.

Introduce sterilant, such as 59% hydrogen peroxide solution, into the vaporizer chamber 12 until the desired concentration is reached, as detected by the sterilant monitor 28 therein. If too much is accidentally introduced, a small portion can be evacuated out. The addition and removal of sterilant can be manipulated until the desired sterilant concentration and pressure is achieved.

The sterilant in the vaporizer chamber 12 will cause the pressure to rise higher than the other portions of the vessel that are currently at 0.2 Torr. Once the valves 20 or 22 isolating the other portions of the vessel are opened, the pressure (and temperature) differentials will immediately force the sterilant into the other portions of the vessel. OPTIONALLY: The valves 22 do not have to be opened or closed simultaneously, allowing for different exposure times for different samples.

At the end of the desired exposure time, evacuate the vessel portions to 0.2 Torr to remove the remaining sterilant before opening the doors to remove the samples. The evacuation time will be less than 30 seconds for the chambers and less than 5 seconds for the Ports. Optionally, plasma may be introduced to enhance the removal of sterilant residual.

Quickly vent the portions of the vessel to allow the filtered air rushing in to "scrub" the surfaces, freeing sterilant that was being held by other materials, and remove the samples/load. Test the biological indicators.

Although described above in connection with particular embodiments of the present invention, it should be understood the descriptions of the embodiments are illustrative of the invention and are not intended to be limiting. Various modifications and applications may occur to those skilled in the art without departing from the scope of the invention as defined in the appended claims.

## Claims

1. A vacuum sterilization chamber (12;14;78) having a sealable doorway, the sealable doorway comprising:
an opening into the chamber (12;14;78);
a door (60;92) for covering the opening;
a hinge (66;74) connecting the door to the chamber;
a latch (64;108) between the door and the chamber; and
a force equalization system at the hinge (66;74) or at the latch (64;108) and which comprises the door (60;92) having a degree of movement relative to the chamber (12;14;78);
**characterised in that** the degree of movement is toward and away from the chamber and **in that** the force equalization system further comprises a biasing member (72;90) urging the door (60;92) toward the chamber (12;14;78).

2. A vacuum sterilization chamber according to claim 1 wherein the force equalization system is at the hinge (66; 34).

3. A vacuum sterilization chamber according to claim 2 wherein the hinge (66) slidably connects to the chamber (12; 14) wherein to allow the degree of movement toward and away from the chamber.

4. A vacuum sterilization chamber according to claim 3 wherein the hinge (74) connects to the chamber (78) via a fastener (82) through an elongated slot (84) on the hinge.

5. A vacuum sterilization chamber according to claim 3 wherein the hinge (66) attaches to the chamber (12; 14) between a pair of low friction plates (68; 70)

6. A vacuum sterilization chamber according to claim 5 wherein the low friction plates (68; 70) comprise PTFE.

7. A vacuum sterilization chamber according to claim 2 wherein the hinge (66; 74) slidably connects to the door (60; 92) wherein to allow the degree of movement toward and away from the chamber.

8. A vacuum sterilization chamber according to claim 1 wherein the biasing member is a spring (72; 90).

9. A vacuum sterilization chamber according to claim 1 and further comprising a seal (109) about the opening between the door (60; 92) and the chamber(12; 14; 78).

10. A vacuum sterilization chamber according to claim 1 wherein the latch (64; 108) is on an opposite side of the opening from the hinge (66; 74).

11. A method of sealing a door (60; 92) to a vacuum sterilization chamber (12; 14; 78) at an opening thereinto, the method comprising the steps of:
closing the door (60; 92) about a hinge (66; 74) between the door and the chamber;
latching the door to the chamber with a latch (64; 108); and
at either the latch (64; 108) or the hinge (46; 74), allowing a degree of freedom of the door relative to the chamber (12; 14; 78); **characterised in that** the degree of freedom is toward and away from the chamber (12;14;78) and **in that** the method further comprises the step of biasing the door (60; 92) toward the chamber (12; 14; 78).

12. A method according to claim 11 wherein the door (60; 92) is allowed a degree of freedom toward and away from the chamber (12; 14; 78) at the hinge (66; 74).

13. A method according to claim 12 wherein the hinge (66) slides relative to the chamber (12; 14)

14. A method according to claim 13 wherein the hinge (66) slides between low friction plates (68; 70).

15. A method according to claim 14 wherein the plates (68; 70) comprise PTFE.

16. A method according to claim 13 wherein the hinge (74) attaches to the chamber (78) via screws (82) through elongated slots (84) through the hinge (74) which allows the hinge to slide relative to the chamber.

17. A method according to claim 12 wherein the hinge (74) attaches to the chamber (78) via a mounting bracket (76) attached to the chamber.

18. A method according to claim 11 wherein the hinge (66) slides relative to the door (60).

19. A method according to claim 11 wherein a spring (72; 90) applies the force to bias the door (60; 92) toward the chamber (12; 14; 78).

20. A method according to claim 11 and further comprising placing a seal (109) about the opening between the door (60; 92) and the chamber (12; 14; 18).

21. A method according to claim 11 wherein the latch (64; 108) is on an opposite side of the door from the hinge (66; 74).

## Patentansprüche

1. Vakuumsterilisationskammer (12; 14; 78) mit einem abdichtbaren Türdurchlass, wobei der abdichtbare Türdurchlass aufweist:
eine Öffnung in die Kammer (12; 14; 78);
eine Tür (60; 92) zum Abdecken der Öffnung;
ein Scharnier (66; 74), das die Tür mit der Kammer verbindet;
einen Riegel (64; 108) zwischen der Tür und der Kammer; und
ein Kraftausgleichssystem an dem Scharnier (66; 74) oder an dem Riegel (64, 108); und die die Tür (60; 92), mit einem Grad der Bewegung relativ zu der Kammer (12; 14; 78) aufweist;
**dadurch gekennzeichnet, dass** der Grad der Bewegung sich auf die Kammer zu und weg von der Kammer bezieht und dass das Kraftausgleichssystem weiter ein vorbelastendes Element (72; 90) aufweist, das die Tür (60; 92) in Richtung auf die Kammer (12; 14; 78) zwingt.

2. Vakuumsterilisationskammer nach Anspruch 1, bei der sich das Kraftausgleichssystem an dem Scharnier (66; 74) befindet.

3. Vakuumsterilisationskammer nach Anspruch 2, bei der das Scharnier (66) verschieblich mit der Kammer (12; 14) verbunden ist, um so den Grad der Bewegung auf die Kammer zu und von ihr weg zu erlauben.

4. Vakuumsterilisationskammer nach Anspruch 3, bei der das Scharnier (74) mit der Kammer (78) über ein Befestigungselement (82) durch einen länglichen Schlitz (34) auf dem Scharnier verbunden ist.

5. Vakuumsterilisationskammer nach Anspruch 3, bei dem das Gelenk (66) an der Kammer (12; 14) zwischen einem Paar von Platten (68; 70) mit geringer Reibung befestigt ist.

6. Vakuumsterilisationskammer nach Anspruch 5, bei der die Platten (68; 70) mit geringer Reibung PTFE aufweisen.

7. Vakuumsterilisationskammer nach Anspruch 2, bei der das Scharnier (66; 74) verschieblich mit der Tür (60; 92) verbunden ist, um so den Grad der Bewegung auf die Kammer zu und von ihr weg zu erlauben.

8. Vakuumsterilisationskammer nach Anspruch 1, bei der das vorbelastende Element eine Feder (72; 90) ist.

9. Vakuumsterilisationskammer nach Anspruch 1 und weiter mit einer Dichtung (109) um die Öffnung zwischen der Tür (60; 92) und der Kammer (12; 14; 78).

10. Vakuumsterilisationskammer nach Anspruch 1, bei der sich der Riegel (64; 108) auf einer entgegengesetzten Seite der Öffnung in Bezug auf das Scharnier (66; 74) befindet.

11. Verfahren zum Abdichten einer Tür (60; 92) in eine Vakuumsterilisationskammer (12; 14; 78) an einer in diese führende Öffnung, wobei das Verfahren die Schritte aufweist:
Schließen der Tür (60; 92) um ein Scharnier (66; 74) zwischen der Tür und der Kammer;
Verriegeln der Tür in die Kammer mit einem Riegel (64; 108); und
entweder an dem Riegel (64; 108) oder an dem Scharnier (66; 74) Zulassen eines Freiheitsgrades der Tür in Bezug auf die Kammer (12; 14; 78),
**dadurch gekennzeichnet, dass** der Freiheitsgrad auf die Kammer (12; 14; 78) zu und weg von ihr besteht und dass das Verfahren weiter den Schritt des Vorbelastens der Tür (60; 92) auf die Kammer ((12; 14; 78) zu aufweist.

12. Verfahren nach Anspruch 11, bei dem der Tür (60; 92) ein Freiheitsgrad in Richtung auf die Kammer (12; 14; 78) zu und weg von ihr an dem Scharnier (66; 74) erlaubt ist.

13. Verfahren nach Anspruch 12, bei dem sich das Scharnier (66) in Bezug auf die Kammer (12; 14) verschiebt.

14. Verfahren nach Anspruch 13, bei dem sich das Scharnier (66) zwischen Platten (68; 70) niedriger Reibung verschiebt.

15. Verfahren nach Anspruch 14, bei dem die Platten (68; 70) PTFE aufweisen.

16. Verfahren nach Anspruch 13, bei dem das Scharnier (74) an der Kammer (78) über Schrauben (82) durch längliche Schlitze (84) durch das Scharnier (74) befestigt ist, was ermöglicht, dass sich das Scharnier in Bezug auf die Kammer verschiebt.

17. Verfahren nach Anspruch 12, bei dem das Scharnier (74) an der Kammer (78) über eine Einbauklammer (76) befestigt ist, die an der Kammer festgelegt ist.

18. Verfahren nach Anspruch 11, bei dem sich das Scharnier (66) relativ zu der Tür (60) verschiebt.

19. Verfahren nach Anspruch 11, bei dem eine Feder (72; 90) die Kraft ausübt, um die Tür (60; 92) auf die Kammer (12; 14; 78) zu vorzubelasten.

20. Verfahren nach Anspruch 11 und weiter mit dem Anbringen einer Dichtung (109) um die Öffnung zwischen der Tür (60; 92) und der Kammer (12; 14; 78).

21. Verfahren nach Anspruch 11, bei dem sich der Riegel (64; 108) auf einer entgegengesetzten Seite der Tür in Bezug auf das Scharnier (66; 74) befindet.

## Revendications

1. Chambre de stérilisation à vide (12 ; 14 ; 78) ayant une porte qui peut être scellée, la porte qui peut être scellée comprenant :
➢ une ouverture dans la chambre (12 ; 14 ; 78) ;
➢ une porte (60 ; 92) destinée à couvrir l'ouverture ;
➢ une charnière (66 ; 74) qui relie la porte à la chambre ;
➢ un verrou (64 ; 108) situé entre la porte et la chambre ; et
➢ un système d'égalisation de force au niveau de la charnière (66 ; 74) ou au niveau du verrou (64 ; 108) et qui comprend le fait que la porte (60 ; 92) présente un degré de déplacement par rapport à la chambre (12 ; 14 ; 78) ;
**caractérisée en ce que** le degré de déplacement se situe en allant vers, et en s'éloignant de, la chambre et **en ce que** le système d'égalisation de force comprend en outre un élément de sollicitation (72 ; 90) qui pousse la porte (60 ; 92) vers la chambre (12 ; 14 ; 78).

2. Chambre de stérilisation à vide selon la revendication 1, dans laquelle le système d'égalisation de force se situe au niveau de la charnière (66 ; 74).

3. Chambre de stérilisation à vide selon la revendication 2, dans laquelle la charnière (66) est reliée de façon coulissante à la chambre (12 ; 14) de manière à permettre le degré de déplacement en allant vers, et en s'éloignant de, la chambre.

4. Chambre de stérilisation à vide selon la revendication 3, dans laquelle la charnière (74) est reliée à la chambre (78) par l'intermédiaire d'un dispositif de fixation (82) à travers une fente allongée (84) située sur la charnière.

5. Chambre de stérilisation à vide selon la revendication 3, dans laquelle la charnière (66) est fixée à la chambre (12 ; 14) entre une paire de plaques à frottement doux (68 ; 70).

6. Chambre de stérilisation à vide selon la revendication 5, dans laquelle les plaques à frottement doux (68 ; 70) comprennent du PTFE.

7. Chambre de stérilisation à vide selon la revendication 2, dans laquelle la charnière (66 ; 74) est reliée de façon coulissante à la porte (60 ; 92) de manière à permettre le degré de déplacement en allant vers, et en s'éloignant de, la chambre.

8. Chambre de stérilisation à vide selon la revendication 1, dans laquelle l'élément de sollicitation est un ressort (72 ; 90).

9. Chambre de stérilisation à vide selon la revendication 1, comprenant en outre un joint d'étanchéité (109) situé autour de l'ouverture entre la porte (60 ; 92) et la chambre (12 ; 14 ; 78).

10. Chambre de stérilisation à vide selon la revendication 1, dans laquelle le verrou (64 ; 108) se situe sur un côté opposé de l'ouverture à partir de la charnière (66 ; 74).

11. Procédé de scellage d'une porte (60 ; 92) sur une chambre de stérilisation à vide (12 ; 14 ; 78) au niveau d'une ouverture donnant accès à l'intérieur, le procédé comprenant les étapes consistant à :
➢ fermer la porte (60 ; 92) autour d'une charnière (66 ; 74) située entre la porte et la chambre ;
➢ verrouiller la porte sur la chambre à l'aide d'un verrou (64 ; 108); et
➢ au niveau du verrou (64 ; 108) ou de la charnière (66 ; 74), permettre un degré de liberté de la porte (60; 92) par rapport à la chambre (12 ; 14 ; 78) ;
**caractérisée en ce que** le degré de liberté se situe en allant vers, et en s'éloignant de, la chambre (12; 14; 78) et **en ce que** le procédé comprend en outre l'étape consistant à solliciter la porte (60 ; 92) vers la chambre (12 ; 14 ; 78).

12. Procédé selon la revendication 11, dans lequel un degré de liberté en allant vers, et en s'éloignant de, la chambre (12 ; 14 ; 78) au niveau de la charnière (66 ; 74), est autorisé à la porte (60 ; 92).

13. Procédé selon la revendication 12, dans lequel la charnière (66) coulisse par rapport à la chambre (12 ; 14).

14. Procédé selon la revendication 13, dans lequel la charnière (66) coulisse entre des plaques à frottement doux (68 ; 70).

15. Procédé selon la revendication 14, dans lequel les plaques (68 ; 70) comprennent du PTFE.

16. Procédé selon la revendication 13, dans lequel la charnière (74) est fixée à la chambre (78) par l'intermédiaire de vis (82) à travers des fentes allongées (84) à travers la charnière (74), ce qui permet à la charnière de coulisser par rapport à la chambre.

17. Procédé selon la revendication 12, dans lequel la charnière (74) est fixée à la chambre (78) par l'intermédiaire d'un support de montage (76) fixé sur la chambre.

18. Procédé selon la revendication 11, dans lequel la charnière (66) coulisse par rapport à la porte (60).

19. Procédé selon la revendication 11, dans lequel un ressort (72 ; 90) applique la force de manière à solliciter la porte (60 ; 92) vers la chambre (12 ; 14 ; 78).

20. Procédé selon la revendication 11, comprenant en outre l'installation d'un joint d'étanchéité (109) situé autour de l'ouverture entre la porte (60 ; 92) et la chambre (12 ; 14 ; 78).

21. Procédé selon la revendication 11, dans lequel le verrou (64 ; 108) se situe sur un côté opposé de la porte à partir de la charnière (66 ; 74).
